(19) Europäisches Patentamt
European Patent Office
Office européen des brevets

(11) **EP 3 403 706 A1**

(12) **DEMANDE DE BREVET EUROPEEN**

(43) Date de publication:
**21.11.2018 Bulletin 2018/47**

(51) Int Cl.:
***B01D 15/18*** *(2006.01)*     ***C07C 7/13*** *(2006.01)*
***C07C 15/08*** *(2006.01)*

(21) Numéro de dépôt: **18169349.0**

(22) Date de dépôt: **25.04.2018**

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**
Etats d'extension désignés:
**BA ME**
Etats de validation désignés:
**KH MA MD TN**

(30) Priorité: **17.05.2017   FR 1754360**

(71) Demandeur: **IFP Energies nouvelles**
**92852 Rueil-Malmaison Cedex (FR)**

(72) Inventeurs:
• **VONNER, Alexandre**
  **69780 MIONS (FR)**
• **LEINEKUGEL LE COCQ, Damien**
  **69600 OULLINS (FR)**
• **LAROCHE, Catherine**
  **69390 VERNAISON (FR)**
• **ETIENNE, Pascal**
  **38780 ESTRABLIN (FR)**

(74) Mandataire: **IFP Energies nouvelles**
**Rond-point de l'échangeur de Solaize**
**BP3**
**69230 Solaize (FR)**

(54) **PROCEDE DE SEPARATION DES XYLENES EN LIT MOBILE SIMULE ET CONDITIONS OPERATOIRES OPTIMISEES POUR LES UNITES TRAITANT DES CHARGES RICHES EN PARAXYLENE**

(57)     La présente invention décrit un procédé de séparation des xylènes en lit mobile simulé apte au traitement de charges riches en paraxylène (supérieur à 25% poids de paraxylène), dans lequel les conditions opératoires sont optimisées au moyen d'une relation spécifique entre le temps de cycle et le débit de désorbant.

EP 3 403 706 A1

**Description**

CONTEXTE DE L'INVENTION

La présente invention concerne le domaine de la séparation du paraxylène au sein des autres isomères en C8 aromatiques. Pour réaliser cette séparation, on utilise une famille de procédés, et de dispositifs associés, connus sous le nom de procédés de séparation en lit mobile simulé (noté en abrégé LMS), ou de séparation en contre-courant simulé (noté en abrégé CCS), ou encore de procédé VARICOL que nous désignerons ci-après par l'appellation générale de procédés de séparation en CCS. Plus précisément, la présente invention a pour objet l'optimisation des conditions opératoires d'une unité donnée par l'obtention du temps de cycle en fonction du débit de désorbant rapporté au débit de paraxylène contenu dans la charge, dans le cas de charges non conventionnelles riches en paraxylène. On entend par charges riches en paraxylène, des charges ayant une teneur en poids de paraxylène supérieure à 25 %, et plus particulièrement comprise entre 25,01 et 60 % poids.

EXAMEN DE L'ART ANTERIEUR

La séparation en CCS est bien connue dans l'état de la technique. En règle générale, un procédé de séparation du paraxylène fonctionnant en contre-courant simulé comporte au moins quatre zones, et éventuellement cinq ou six, chacune de ces zones étant constituée par un certain nombre de lits successifs, et chaque zone étant définie par sa position comprise entre un point d'alimentation et un point de soutirage. Typiquement, une unité CCS pour la production de paraxylène est alimentée par au moins une charge F à fractionner (contenant le paraxylène et les autres isomères en C8 aromatiques) et un désorbant D, parfois appelé éluant (généralement du paradiéthylbenzène ou du toluène), et l'on soutire de ladite unité au moins un raffinat R contenant les isomères du paraxylène et du désorbant et un extrait E contenant le paraxylène et du désorbant.

Les procédés de séparation des xylènes en lit mobile simulé fonctionnent classiquement avec des réglages qui visent à maximiser la productivité, au prix d'un flux de désorbant important, et donc d'un coût d'utilités élevé (rebouillage des colonnes de distillations, pompes...)

La présente invention concerne le domaine des faibles taux de désorbant, c'est-à-dire le domaine des faibles ratio entre débit de désorbant et débit de charge, où un compromis est trouvé entre la productivité et la réduction du débit de désorbant, et donc la réduction des coûts opératoires.

Nous n'avons pas détecté d'art antérieur portant spécifiquement sur le lien entre le débit de désorbant et le temps de cycle, c'est-à-dire le temps pendant lequel les injections et soutirages de l'unité évoluent le long de la colonne jusqu'à retrouver leur position initiale. Et à fortiori pas d'enseignement de ce type pour les charges à fortes teneurs en paraxylène qui concernent la présente invention.

DESCRIPTION SOMMAIRE DE L'INVENTION

La présente invention décrit un ensemble de réglages optimisés d'une unité de séparation et de purification en lit mobile simulé du paraxylène contenu dans une charge d'isomères en C8, la teneur en paraxylène dans la charge étant comprise entre 25,01 et 60 % poids, et la teneur en éthylbenzène étant comprise entre 2 et 15 % poids. Par réglage optimisé, il est entendu que les performances de pureté et rendement, ainsi que la productivité maximale en paraxylène, sont atteintes pour un taux de désorbant donné.

L'unité de séparation des xylènes en lit mobile simulé utilise au moins une colonne dont le nombre de lits est compris entre 4 et 24, préférentiellement compris entre 6 et 18, et de manière encore préférée compris entre 8 et 15.

On peut définir la configuration de l'unité en définissant le nombre moyen de lit pour la zone j (j étant compris entre 1 et 4) Nzj par rapport au nombre total de lits dans l'ensemble de l'unité N_total, de la manière suivante :

$$Nz1 = (N\_total * 5 / 24) * ( 1 \pm 0{,}2 )$$

$$Nz2 = (N\_total * 9 / 24) * ( 1 \pm 0{,}2 )$$

$$Nz3 = (N\_total * 7 / 24) * ( 1 \pm 0{,}2 )$$

$$Nz4 = (N\_total * 3 / 24) * ( 1 \pm 0{,}2 )$$

Les 4 zones chromatographiques étant définies de la manière suivante :

- Zone 1 : zone de désorption du paraxylène, comprise entre l'injection du désorbant D et le prélèvement de l'extrait E ;
- Zone 2 : zone de désorption des isomères du paraxylène, comprise entre le prélèvement de l'extrait E et l'injection de la charge à fractionner F ;
- Zone 3 : zone d'adsorption du paraxylène, comprise entre l'injection de la charge et le soutirage du raffinat R ;
- Zone 4 : zone située entre le soutirage de raffinat R et l'injection du désorbant D.

Le désorbant utilisé dans le cadre de la présente invention est le paradiéthylbenzène.

Les réglages optimisés du lit mobile simulé sont décrits sous forme de couples taux de désorbant pour un temps de cycle pondéré.

Le taux de désorbant est le ratio débit de désorbant sur débit de paraxylène contenu dans la charge.

Le temps de cycle est l'intervalle de temps séparant deux injections de désorbant au même endroit dans l'adsorbeur. Il est appelé ici pondéré quand il est multiplié par le facteur $\dfrac{\mu}{\sigma^2} \cdot \dfrac{1}{\varepsilon_i.L_{lit}}$ ;

Le rapport $\dfrac{\mu}{\sigma^2}$ exprimé en 1/s est déterminé par une expérience de perçage telle que décrite plus précisément dans la description détaillée de l'invention qui suit.

Il est important de s'assurer que la mesure de $\sigma^2$ est représentative du transfert de matière global au sein de l'adsorbant, et non pas de la dispersion liée à l'hydrodynamique dans la colonne et dans les lignes de l'outil de test. Pour ce faire, un test de perçage doit être réalisé dans les mêmes conditions que le test effectué avec une colonne remplie d'adsorbant, mais en remplissant la colonne avec des billes de verre de diamètre comparable avec le diamètre de l'adsorbant. La mesure de $\sigma^2$ obtenu en présence d'adsorbant doit être au moins 10 fois supérieure, de préférence 30 fois supérieure à la mesure $\sigma^2_{blanc}$ obtenue lors de ce test en absence d'adsorbant.

$L_{lit}$ représente la longueur d'un lit exprimée en mètres et $\varepsilon_i$ la porosité interstitielle moyenne d'un lit d'adsorbant.

Le temps de cycle (intervalle de temps séparant deux injections de désorbant au même endroit dans la colonne) optimisé est déterminé à partir du temps de cycle pondéré par l'intermédiaire du facteur :

$$\frac{\mu}{\sigma^2} \cdot \frac{1}{\varepsilon_i.L_{lit}}$$

connaissant la longueur d'un lit ($L_{lit}$) et sa porosité interstitielle ($\varepsilon_i$), le ratio débit de désorbant sur débit de paraxylène, donc le débit de désorbant optimal recherché ($Q_D$) est déterminé suivant la gamme de concentration en paraxylène (PX) dans la charge au moyen des trois tableaux suivants, $Q_{PX}$ désignant le débit entrant de paraxylène :

**Tableau 1**

| Paraxylène PX dans la charge : 25 % < %$_{PX,charge}$ ≤ 30% | | |
|---|---|---|
| | $t\_cycle\ pond$ (1/m) | $Q_D/Q_{PX}$ |
| Réglage n°1 | 320 +/- 25 | 5,30 +/- 0,55 |
| Réglage n°2 | 345 +/- 25 | 4,85 +/- 0,50 |
| Réglage n°3 | 370 +/- 25 | 4,50 +/- 0,45 |
| Réglage n°4 | 395 +/- 25 | 4,20 +/- 0,45 |
| Réglage n°5 | 425 +/- 25 | 4,00 +/- 0,45 |
| Réglage n°6 | 465 +/- 25 | 3,75 +/- 0,40 |
| Réglage n°7 | 510 +/-25 | 3,60 +/- 0,40 |
| Réglage n°8 | 560 +/- 30 | 3,55 +/- 0,40 |

**Tableau 2**

| Paraxylène PX dans la charge : 30 % < %$_{PX,charge}$ ≤ 45 % | | |
|---|---|---|
| | t_cycle pond (1/m) | $Q_D/Q_{PX}$ |
| Réglage n°1 | 325 +/- 25 | 4,40 +/- 0,45 |
| Réglage n°2 | 345 +/- 25 | 4,00 +/- 0,40 |
| Réglage n°3 | 365 +/- 25 | 3,75 +/- 0,40 |
| Réglage n°4 | 385 +/- 25 | 3,50 +/- 0,40 |
| Réglage n°5 | 415 +/- 25 | 3,30 +/- 0,35 |
| Réglage n°6 | 445 +/- 25 | 3,15 +/- 0,35 |
| Réglage n°7 | 485 +/- 25 | 3,00 +/- 0,35 |
| Réglage n°8 | 530 +/- 25 | 2,90 +/- 0,35 |
| Réglage n°9 | 580 +/- 30 | 2,80 +/- 0,30 |
| Réglage n°10 | 640 +/- 30 | 2,80 +/- 0,30 |

**Tableau 3**

| Paraxylène PX dans la charge : 45 % < %$_{PX,charge}$ ≤ 60 % | | |
|---|---|---|
| | t_cycle pond (1/m) | $Q_D/Q_{PX}$ |
| Réglage n°1 | 315 +/- 25 | 4,10 +/- 0,50 |
| Réglage n°2 | 335 +/- 25 | 3,65 +/- 0,45 |
| Réglage n°3 | 350 +/- 25 | 3,25 +/- 0,35 |
| Réglage n°4 | 370 +/- 25 | 3,05 +/- 0,35 |
| Réglage n°5 | 400 +/- 25 | 2,80 +/- 0,35 |
| Réglage n°6 | 440 +/- 25 | 2,65 +/- 0,30 |
| Réglage n°7 | 480 +/- 25 | 2,55 +/- 0,30 |
| Réglage n°8 | 525 +/- 25 | 2,45 +/- 0,30 |
| Réglage n°9 | 580 +/- 30 | 2,40 +/- 0,30 |
| Réglage n°10 | 640 +/- 30 | 2,35 +/- 0,30 |

Les conditions opératoires du procédé de séparation des xylènes en lit mobile simulé selon l'invention sont les suivantes :

- la température opératoire est généralement comprise entre 100°C et 250°C, préférentiellement entre 120°C et 180°C, et
- la pression est comprise entre la pression de bulle du mélange de xylènes constituant la charge et 3 MPa.

La teneur en eau dans la charge du procédé de séparation des xylènes en lit mobile simulé selon l'invention est généralement comprise entre 70 et 140 ppm, et préférentiellement comprise entre 80 et 120 ppm.

Le désorbant utilisé dans le procédé de séparation des xylènes selon la présente l'invention est le paradiéthylbenzène.

DESCRIPTION DETAILLEE DE L'INVENTION

Le problème que cherche à résoudre la présente invention est celui de régler de manière optimale le procédé dans une gamme basse de taux de désorbant, pour réduire le débit de « tourne en rond », et optimiser les coûts d'utilité (OPEX) du procédé. Par réglage optimal, il est entendu un réglage permettant d'obtenir un débit minimal de désorbant pour un niveau de productivité donné, tout en garantissant les niveaux de pureté et de rendement requis : typiquement une pureté du paraxylène supérieure à 99,5%, de manière préférée supérieure à 99,6%, et de manière encore préférée supérieure à 99,7%, et un rendement en paraxylène supérieur à 95%, de manière préférée supérieur à 96%, et de manière encore préférée supérieur à 97%. En l'état de l'art actuel, il n'existe pas de méthode de réglage optimal de l'unité de séparation des xylènes.

L'invention concerne un procédé de production de paraxylène à haut niveau de pureté, à partir d'une charge F dite non conventionnelle, riche en paraxylène, c'est-à-dire comprenant entre 25,01% et 60% poids de paraxylène, associé à ses autres isomères en C8 aromatiques.

Selon une caractéristique du procédé, l'adsorbant du procédé selon l'invention est un adsorbant zéolitique à base de cristaux de zéolite X et de phase non zéolithique, de manière préférée une zéolithe de type faujasite échangée au baryum ou échangée au baryum et au potassium.

La séparation par LMS du paraxylène de pureté commerciale, typiquement à au moins 99,7% poids, est réalisée industriellement dans les dispositifs LMS comprenant n lits d'adsorbants, n pouvant être compris entre 4 et 24, de préférence entre 6 et 18 lits, et de manière encore plus préférée entre 8 et 15 lits.

Le nombre de lits est ajusté de manière à ce que chaque lit ait de préférence une hauteur comprise entre 70 cm et 1,40 m.

La configuration de chaque zone, c'est-à-dire le nombre moyen de lits par zone de l'unité selon l'invention, peut-être à nombre de lits fixe (les décalages des différents points d'injection ou de soutirage étant alors simultanés) ou variable. Dans ce dernier cas, les décalages des 2 points d'injection et des 2 points de soutirage ne sont pas simultanés, de manière à obtenir des nombres de lits par zone qui ne sont pas entiers en moyenne au cours d'un cycle comme l'enseigne le brevet FR2785196. On peut définir la configuration de l'unité en définissant le nombre moyen de lit pour la zone j (j étant compris entre 1 et 4) Nzj par rapport au nombre total de lits dans l'ensemble de l'unité N_total, de la manière suivante. Dans ces expressions le premier indice z est le nombre de lits dans la zone considérée, et le second indice j variant de 1 à 4, représente la zone considérée.

$$Nz1 = (N\_total * 5/24) * (1 \pm 0,2)$$

$$Nz2 = (N\_total * 9/24) * (1 \pm 0,2)$$

$$Nz3 = (N\_total * 7/24) * (1 \pm 0,2)$$

$$Nz4 = (N\_total * 3/24) * (1 \pm 0,2)$$

Il est possible par application des formules précédentes de tomber sur des nombres de lits non entiers. Cela ne pose pas de problème dans le contexte de la présente invention, car il existe une variante du procédé Eluxyl dite « varicol » qui autorise un tel fonctionnement.

Les 4 zones chromatographiques sont définies de manière générale de la manière suivante :

- Zone 1 : zone de désorption du paraxylène, comprise entre l'injection du désorbant D et le prélèvement de l'extrait E ;
- Zone 2 : zone de désorption des isomères du paraxylène, comprise entre le prélèvement de l'extrait E et l'injection de la charge à fractionner F ;
- Zone 3 : zone d'adsorption du paraxylène, comprise entre l'injection de la charge et le soutirage du raffinat R ;
- Zone 4 : zone située entre le soutirage de raffinat R et l'injection du désorbant D.

De manière avantageuse, le temps de cycle, correspondant au temps entre deux injections de désorbant sur un lit donné, est compris entre 3 et 50 min, et de manière préférée entre 5 et 45 min.

De manière avantageuse, le procédé de séparation des xylènes opère à une température de 175°C à +/- 10°C, et à une pression comprise entre la pression de bulle des xylènes à la température du procédé et 3 MPa.

De manière avantageuse, le taux de recyclage est compris entre 2,0 et 8, de préférence 2,5 à 5. On définit le taux de recyclage comme le rapport entre le débit moyen s'écoulant dans les différents lits de l'adsorbeur sur le débit d'injection de charge dans cet adsorbeur.

La teneur en eau en phase liquide est maintenue à une teneur comprise entre 80 et 120 ppm (poids).

Dans le procédé de séparation du paraxylène selon l'invention, le désorbant est le paradiéthylbenzène.

Outre une capacité d'adsorption élevée et de bonnes propriétés de sélectivité vis-à-vis de l'espèce à séparer du mélange réactionnel, l'adsorbant doit présenter de bonnes propriétés de transfert de matière afin de garantir un nombre de plateaux théoriques suffisant pour réaliser une séparation efficace des espèces en mélange, comme l'indique Ruthven dans l'ouvrage intitulé «Principles of Adsorption and Adsorption Processes » qu'on peut traduire par «Principes de l'Adsorption et des Procédés d'Adsorption », John Wiley & Sons, (1984), pages 326 et 407.

Pour estimer le transfert de matière global d'un lit d'adsorbant, une technique simple consiste à réaliser une expérience chromatographique, dans laquelle l'adsorbant conditionné dans une colonne, est soumis à une perturbation de la concentration en entrée de colonne. Cette technique est décrite dans le document suivant :
Silva, M. S. P.; Moreira, M. A.; Ferreira, A. F. P.; Santos, J. C.; Silva, V. M. T. M.; Sá Gomes, P.; Minceva, M.; Mota, J. P. B.; Rodrigues, A. E. Adsorbent Evaluation Based on Experimental Breakthrough Curves: Separation of p- Xylene from C8 Isomers.Chem. Eng. Technol. 2012, 35, 1777-1785.

L'analyse du front de concentration en sortie de colonne en fonction du temps, noté c(t), en réponse à la perturbation de concentration en entrée, permet d'estimer les propriétés d'adsorption et de transfert de matière global.

Lorsque la perturbation en entrée est un échelon de concentration, l'expérience réalisée est connue sous le nom de « perçage », et le front de concentration obtenu en sortie de colonne en fonction du temps est appelé « courbe de perçage ».

Dans l'ouvrage intitulé « Diffusion in Nanoporous », qu'on peut traduire par « Diffusion dans les nanopores », de Kärger, Ruthven et Theodorou, ed. Wiley (2012), l'analyse de la réponse expérimentale d'une colonne chromatographique par la méthode des moments est décrite au chapitre 14, pages 464-465.

Dans le cas d'un perçage, réponse à un échelon de concentration $c_0$, les premiers et seconds moments du front de concentration c(t) en sortie de colonne en fonction du temps, sont donnés par les expressions suivantes :

$\mu$ , le premier moment de la courbe de perçage, c'est-à-dire le temps moyen de sortie $\bar{t}$ du front de concentration de la colonne chromatographique :

$$\mu = \bar{t} = \int_0^\infty \left(1 - \frac{c}{c_0}\right) dt$$

$\sigma^2$, le second moment centré de la courbe de perçage, qui traduit la dispersion du front de concentration :

$$\sigma^2 = 2 \int_0^\infty \left(1 - \frac{c}{c_0}\right) t\, dt - \mu^2$$

c(t) est la fonction concentration en fonction du temps consécutive à la perturbation $c_0$ introduite en entrée.

Il est important de s'assurer que la mesure de $\sigma^2$ est représentative du transfert de matière global au sein de l'adsorbant et non pas de la dispersion liée à l'hydrodynamique dans la colonne et dans les lignes de l'outil de test. Pour ce faire, un test de perçage doit être réalisé dans les mêmes conditions que le test effectué avec une colonne remplie d'adsorbant, mais en remplissant la colonne avec des billes de verre de diamètre approximativement égal au diamètre des particules de l'adsorbant. La mesure de $\sigma^2$ obtenu en présence d'adsorbant doit être au moins 10 fois supérieure, de préférence 30 fois supérieure à la mesure $\sigma^2_{blanc}$ obtenue lors de ce test en absence d'adsorbant. On entend par « approximativement égal » égal à plus ou moins 10%.

Le temps de cycle est un des paramètres opératoires, à l'instar des débits d'injection de charge, de désorbant, et des débits de soutirage d'extrait et de raffinat, qui sont définis au cas par cas par les opérateurs du procédé. En l'état de l'art actuel, il n'existe pas de méthode de réglage systématique.

La présente invention décrit la relation entre :

- le taux de désorbant, exprimé comme le ratio du débit de désorbant $Q_D$ sur le débit de paraxylène dans la charge $Q_{PX}$,

- $t_{cycle}$ le temps de cycle minimal du procédé, pondéré par le ratio du premier moment du perçage $\mu$, sur le produit du second moment centré du perçage $\sigma^2$, de $L_{lit}$ la longueur d'un lit et de $\varepsilon_i$ la porosité de l'empilement de solide adsorbant :

$$t_{cycle,pondéré} = t_{cycle} \cdot \frac{\mu}{\sigma^2} \cdot \frac{1}{\varepsilon_i \cdot L_{lit}}$$

Le temps de cycle pondéré est donc relié au temps de cycle par un groupe de paramètres qui sont les premier et second moments de la courbe de perçage obtenus de manière expérimentale, et deux grandeurs du lit : sa longueur ($L_{lit}$) et sa porosité interstitielle ($\varepsilon_i$). Ce temps de cycle pondéré a alors comme dimension l'inverse d'une longueur, on l'exprime donc en 1/m.

L'invention ainsi décrite est valable quelles que soient les propriétés de transfert de matière du solide adsorbant et la configuration du procédé, qui sont renseignés dans le facteur de pondération du temps de cycle.

Les premier et second moments du perçage sont déterminés pour une vitesse superficielle donnée du mélange injecté lors de l'expérience de perçage, égale à 1,30 cm/s +/- 0,05 cm/s dans les conditions de température du test, soit 175°C, et pour une longueur donnée de colonne égale à 1,00 m +/- 0,01 m.

Les réglages optimisés décrits par l'invention sont présentés dans les 3 tableaux suivants, suivant la gamme de concentration de la charge en paraxylène :

**Tableau 1 : 25 % < Paraxylène PX dans la charge $\leq$ 30 %**

| 25 % < Paraxylène PX dans la charge $\leq$ 30 % | | |
|---|---|---|
| | $t\_cycle\,pond$ (1/m) | $Q_D/Q_{PX}$ |
| Réglage n°1 | 320 +/- 25 | 5,30 +/- 0,55 |
| Réglage n°2 | 345 +/- 25 | 4,85 +/- 0,50 |
| Réglage n°3 | 370 +/- 25 | 4,50 +/- 0,45 |
| Réglage n°4 | 395 +/- 25 | 4,20 +/- 0,45 |
| Réglage n°5 | 425 +/- 25 | 4,00 +/- 0,45 |
| Réglage n°6 | 465 +/- 25 | 3,75 +/- 0,40 |
| Réglage n°7 | 510 +/- 25 | 3,60 +/- 0,40 |
| Réglage n°8 | 560 +/- 30 | 3,55 +/- 0,40 |

**Tableau 2 : 30 % < Paraxylène PX dans la charge $\leq$ 45 %**

| 30 % < Paraxylène PX dans la charge $\leq$ 45 % | | |
|---|---|---|
| | $t\_cycle\,pond$ (1/m) | $Q_D/Q_{PX}$ |
| Réglage n°1 | 325 +/- 25 | 4,40 +/- 0,45 |
| Réglage n°2 | 345 +/- 25 | 4,00 +/- 0,40 |
| Réglage n°3 | 365 +/- 25 | 3,75 +/- 0,40 |
| Réglage n°4 | 385 +/- 25 | 3,50 +/- 0,40 |
| Réglage n°5 | 415 +/- 25 | 3,30 +/- 0,35 |
| Réglage n°6 | 445 +/- 25 | 3,15 +/- 0,35 |
| Réglage n°7 | 485 +/- 25 | 3,00 +/- 0,35 |
| Réglage n°8 | 530 +/- 25 | 2,90 +/- 0,35 |
| Réglage n°9 | 580 +/- 30 | 2,80 +/- 0,30 |
| Réglage n°10 | 640 +/- 30 | 2,80 +/- 0,30 |

**Tableau 3 : 45 % < Paraxylène PX dans la charge ≤ 60 %**

| 45% < Paraxylène PX dans la charge ≤ 60 % | | |
|---|---|---|
| | $t\_cycle\ pond$ (1/m) | $Q_D/Q_{PX}$ |
| Réglage n°1 | 315 +/- 25 | 4,10 +/- 0,50 |
| Réglage n°2 | 335 +/- 25 | 3,65 +/- 0,45 |
| Réglage n°3 | 350 +/- 25 | 3,25 +/- 0,35 |
| Réglage n°4 | 370 +/- 25 | 3,05 +/- 0,35 |
| Réglage n°5 | 400 +/- 25 | 2,80 +/- 0,35 |
| Réglage n°6 | 440 +/- 25 | 2,65 +/- 0,30 |
| Réglage n°7 | 480 +/- 25 | 2,55 +/- 0,30 |
| Réglage n°8 | 525 +/- 25 | 2,45 +/- 0,30 |
| Réglage n°9 | 580 +/- 30 | 2,40 +/- 0,30 |
| Réglage n°10 | 640 +/- 30 | 2,35 +/- 0,30 |

Les points définis dans les 3 tableaux ci-dessus décrivent des réglages optimaux, au sens où la productivité en paraxylène est maximale pour le taux de désorbant considéré.

- Les réglages à moindre taux de désorbant, ou à un temps de cycle pondéré plus petit, ne permettent pas d'atteindre les cibles de pureté et de rendement du procédé.

- Les réglages à plus grand taux de désorbant, ou à un temps de cycle pondéré supérieur, permettent d'atteindre les performances, mais, soit la productivité en paraxylène est inférieure à sa valeur optimale, soit le taux de désorbant n'est pas aussi petit que possible pour cette productivité. On cherche donc à se placer aussi près que possible de ces réglages optimisés qui sont l'objet de l'invention.

EXEMPLES SELON L'INVENTION

L'invention sera mieux comprise à la lecture des trois exemples qui suivent. Le premier correspond à une unité réglée de manière optimale selon l'invention, le second à une unité « sous performante » réglée au même taux de désorbant, et le troisième à une unité « sur-performante » réglée au même taux de désorbant.
Un test de perçage (chromatographie frontale) est réalisé sur les adsorbants dans le but d'évaluer le second moment réduit $\dfrac{\sigma^2}{2\mu^2}$ en fonction de la vitesse superficielle du fluide injecté. Le perçage consiste à injecter en continu, à travers une colonne remplie d'adsorbant, de la charge contenant un ou des composés qui vont y être adsorbés. La colonne est préalablement saturée par un solvant. La colonne utilisée a une longueur de 1,00 mètre et un diamètre interne de 0,77 cm et la quantité de solide adsorbant pour ce test est d'environ 40 g.
Le mode opératoire pour obtenir les courbes de perçage est le suivant :

◦ Remplissage de la colonne par le solide adsorbant et mise en place dans le banc de test.

◦ Remplissage par le solvant à température ambiante.

◦ Montée progressive de la température d'adsorption sous flux de solvant (consigne de débit à température ambiante de 5 cm$^3$/min).

◦ Injection de solvant à 30 cm$^3$/min (consigne de débit à température ambiante) lorsque la température d'adsorption est atteinte.

◦ Permutation solvant/charge pour injecter la charge (consigne de débit à température ambiante de 30 cm$^3$/min).

◦ Collecte en continu de l'effluent du perçage dans des vials fermés au fur et à mesure et analyse par chromatographie en phase gazeuse de l'effluent collecté dans les vials.

◦ L'injection de la charge est maintenue un temps suffisant pour atteindre l'équilibre thermodynamique (c'est-à-dire jusqu'à ce que la concentration en solvant dans l'effluent soit nulle).

Pour l'obtention de la courbe de perçage, le solvant utilisé est l'orthoxylène. La charge utilisée est constituée uniquement de métaxylène.

Le test est réalisé avec une température d'adsorption de 175°C. La pression est suffisante pour que la charge reste en phase liquide, soit 1 MPa.

Deux tests sont réalisés : un test avec la colonne remplie de bille de verre de granulométrie 400-600 $\mu$m à partir duquel est évalué le $\sigma^2_{blanc}$, et un second test avec la même colonne (ou une colonne identique) remplie d'adsorbant de granulométrie 400-600 $\mu$m, à partir duquel sont évalués le premier $\mu$ et le second moment centré $\sigma^2$.

Les résultats sont rassemblés dans le tableau 4 ci-dessous :

**Tableau 4 : Détermination du second moment à partir d'une expérience de perçage**

| Vitesse superficielle liquide | 1,30 | cm/s |
|---|---|---|
| $\sigma^2_{blanc}$ | 0,001 | min$^2$ |
| 1$^{er}$ moment $\mu$ | 0,98 | min |
| $\sigma^2$ | 0,129 | min$^2$ |
| $\sigma^2/\sigma^2_{blanc}$ | 129 | |
| $\sigma^2/2.\mu^2$ | 0,067 | |
| $\mu/\sigma^2$ | 7,69 | 1/min |

**Exemple 1 (unité réglée de manière optimale) :**

On considère une unité de lit mobile simulé constituée de 15 lits, chacun de longueur 1,24 m, de porosité interstitielle 39,6%, et de rayon interne 1,05 m, avec une injection de charge, une injection de désorbant, un soutirage d'extrait et un soutirage de raffinat.

L'adsorbant considéré est un solide zéolitique de type BaX, caractérisé au perçage suivant la méthode précédemment décrite par un ratio $\mu/\sigma^2$ = 7,69 1/min.

Le désorbant est du paradiéthylbenzène. La température est de 175 °C, la pression de 15 bars. La teneur en eau est de 95 ppm (poids).

La charge à séparer est composée de 50 % de paraxylène, de 17,3 % d'orthoxylène, 22,7 % de métaxylène et 10 % d'éthylbenzène.

Les décalages des différents points d'injection ou de soutirage sont simultanés. Les lits sont répartis en 4 zones chromatographiques selon la configuration 3 / 5 / 5 / 2.

Les débits d'injection de charge et de désorbant sont les suivants :

- 0,393 m$^3$.min$^{-1}$ pour la charge,
- 0,591 m$^3$.min$^{-1}$ pour le désorbant.

De plus, le débit de zone 4 est de 1,527 m$^3$.min$^{-1}$, et le débit de soutirage d'extrait est de 0,377 m$^3$.min$^{-1}$. Le temps de cycle pondéré est de 396,1 m$^{-1}$.

On obtient par simulation une pureté de paraxylène de 99,78 % et un rendement en paraxylène de 97,7 % avec une productivité de 146,0 kg$_{PX}$.h$^{-1}$.m$^{-3}$.

Ce niveau de performances en termes de pureté et de rendement en paraxylène est conforme aux cibles du procédé, et sera pris comme référence pour les exemples suivants.

**Exemple 2 (unité « sous-performante») :**

A partir de l'unité considérée comme réglée de manière optimale en terme de pureté et de rendement en paraxylène, présentée dans l'exemple 1, on veut réduire le temps de cycle pour améliorer la productivité, en conservant le même taux de désorbant.

On considère toujours une unité de lit mobile simulé constituée de 15 lits, de longueur 1,24 m, de porosité interstitielle 39,6%, et de rayon interne 1,05 m, avec une injection de charge, une injection de désorbant, un soutirage d'extrait et un soutirage de raffinat.

L'adsorbant considéré est un solide zéolitique de type BaX, caractérisé au perçage suivant la méthode précédemment décrite par un ratio $\mu/\sigma^2$ = 7,69 1/min.

Le désorbant est du paradiéthylbenzène. La température est de 175 °C, la pression de 15 bars. La teneur en eau est de 95 ppm (poids).

La charge est composée de 50 % de paraxylène, de 17,3 % d'orthoxylène, 22,7 % de métaxylène et 10 % d'éthylbenzène.

Les décalages des différents points d'injection ou de soutirage sont simultanés. Les lits sont répartis en 4 zones chromatographiques selon la configuration 3 / 5 / 5 / 2.

Les débits d'injection de charge et de désorbant sont les suivants :

- 0,431 m$^3$.min$^{-1}$ pour la charge,
- 0,648 m$^3$.min$^{-1}$ pour le désorbant.

De plus, le débit de zone 4 est de 1,676 m$^3$.min$^{-1}$, et le débit de soutirage d'extrait est de 0,414 m$^3$.min$^{-1}$. Le temps de cycle pondéré est de 360,8 m$^{-1}$.

On obtient par simulation une pureté de paraxylène de 99,69 % et un rendement en paraxylène de 97,3 % avec une productivité de 159,6 kg$_{PX}$.h$^{-1}$.m$^{-3}$. L'unité ainsi réglée est « sous-performante » par rapport aux cibles de pureté et de rendement, qui sont respectivement de 99,78 % et 97,7 %.

**Exemple 3 (unité « sur-performante»)**

A partir de l'unité considérée dans l'exemple 1, on veut augmenter le temps de cycle pour améliorer les performances en termes de pureté et de rendement en paraxylène, en conservant le même taux de désorbant.

On considère toujours une unité de lit mobile simulé constituée de 15 lits, de longueur 1,24 m, de porosité interstitielle 39,6%, et de rayon interne 1,05 m, avec une injection de charge, une injection de désorbant, un soutirage d'extrait et un soutirage de raffinat.

L'adsorbant considéré est un solide zéolitique de type BaX, caractérisé au perçage suivant la méthode précédemment décrite par un ratio $\mu/\sigma^2$ = 7,69 1/min.

Le désorbant est du paradiéthylbenzène. La température est de 175 °C, la pression de 15 bars. La teneur en eau est de 95 ppm (poids).

La charge est composée de 50 % de paraxylène, de 17,3 % d'orthoxylène, 22,7 % de métaxylène et 10 % d'éthylbenzène.

Les décalages des différents points d'injection ou de soutirage sont simultanés. Les lits sont répartis en 4 zones chromatographiques selon la configuration 3 / 5 / 5 / 2.

Les débits d'injection de charge et de désorbant sont les suivants :

- 0,361 m$^3$.min$^{-1}$ pour la charge,

- 0,542 m$^3$.min$^{-1}$ pour le désorbant.

De plus, le débit de zone 4 est de 1,402 m$^3$.min$^{-1}$, et le débit de soutirage d'extrait est de 0,346 m$^3$.min$^{-1}$. Le temps de cycle pondéré est de 431,4 m$^{-1}$.

On obtient par simulation une pureté de paraxylène de 99,84 % et un rendement en paraxylène de 97,9 % avec une productivité de 134,4 kg$_{PX}$.h$^{-1}$.m$^{-3}$. L'unité ainsi réglée est sur-performante par rapport aux cibles de pureté et de rendement, qui sont respectivement de 99,78 % et 97,7 %.

Ces exemples illustrent bien l'intérêt du réglage du procédé selon l'invention, pour déterminer le temps de cycle qui permet d'obtenir les performances optimales pour un taux de désorbant donné, à savoir simultanément les niveaux cibles de pureté et rendement en paraxylène, ainsi que la productivité maximale pour ces réglages.

**Revendications**

1. Procédé de séparation des xylènes d'une charge (F) à fractionner en lit mobile simulé en vue d'optimiser la pureté et le rendement en paraxylène, la teneur en poids de paraxylène dans la charge étant comprise entre 25,01 et 60 % poids, l'unité mettant en oeuvre ledit procédé utilisant un nombre de lits compris entre 4 et 24, préférentiellement compris entre 6 et 18, et de manière encore préférée compris entre 8 et 15, et la répartition des lits dans les différentes zones étant donnée par la formule générale suivante, valable quelle que soit le nombre total de lits (N_total), l'indice z désignant le nombre de lits dans la zone considérée, et le second indice étant celui de la zone considérée :

$$Nz1 = (N\_total * 5/24) * (1 \pm 0{,}2)$$

$$Nz2 = (N\_total * 9/24) * (1 \pm 0{,}2)$$

$$Nz3 = (N\_total * 7/24) * (1 \pm 0{,}2)$$

$$Nz4 = (N\_total * 3/24) * (1 \pm 0{,}2)$$

les 4 zones chromatographiques étant définies de la manière suivante :

- Zone 1 : zone de désorption du paraxylène, comprise entre l'injection du désorbant (D) et le prélèvement de l'extrait (E) ;
- Zone 2 : zone de désorption des isomères du paraxylène, comprise entre le prélèvement de l'extrait (E) et l'injection de la charge (F) à fractionner ;
- Zone 3 : zone d'adsorption du paraxylène, comprise entre l'injection de la charge et le soutirage du raffinat (R) ;
- Zone 4 : zone située entre le soutirage de raffinat (R) et l'injection du désorbant (D),

- le temps de cycle (intervalle de temps séparant deux injections de désorbant au même endroit dans la colonne),optimisé étant déterminé à partir du temps de cycle pondéré par l'intermédiaire du facteur correctif :

$$\frac{\mu}{\sigma^2} \cdot \frac{1}{\varepsilon_i . L_{lit}}$$

- le paramètre $\frac{\mu}{\sigma^2}$ étant lui-même déterminé par une expérience de perçage, connaissant la longueur d'un lit ($L_{lit}$) et sa porosité interstitielle ($\varepsilon_i$), la mesure de $\sigma^2$ obtenu en présence d'adsorbant devant être au moins 10 fois supérieure, de préférence 30 fois supérieure, à la mesure $\sigma^2_{blanc}$ obtenue lors d'un test réalisé en absence d'adsorbant, avec des billes de verre de diamètre approximativement égal à celui des particules d'adsorbant,

- le ratio débit de désorbant sur débit de paraxylène (QD/QPX), donc le débit de désorbant optimal est déterminé au moyen des 3 tableaux suivants en fonction de la teneur en paraxylène dans la charge :

**Tableau 1 : Paraxylène PX dans la charge : 25 % < %$_{PX,charge} \leq$ 30 %**

| 25 % < Paraxylène PX dans la charge $\leq$ 30 % | | |
|---|---|---|
| | $t\_cycle\ pond$ (1/m) | $Q_D/Q_{PX}$ |
| Réglage n°1 | 320 +/- 25 | 5,30 +/- 0,55 |
| Réglage n°2 | 345 +/- 25 | 4,85 +/- 0,50 |
| Réglage n°3 | 370 +/- 25 | 4,50 +/- 0,45 |
| Réglage n°4 | 395 +/- 25 | 4,20 +/- 0,45 |
| Réglage n°5 | 425 +/- 25 | 4,00 +/- 0,45 |
| Réglage n°6 | 465 +/- 25 | 3,75 +/- 0,40 |
| Réglage n°7 | 510 +/- 25 | 3,60 +/- 0,40 |
| Réglage n°8 | 560 +/- 30 | 3,55 +/- 0,40 |

**Tableau 2 : Paraxylène PX dans la charge : 30 % < %$_{PX,charge}$ ≤ 45 %**

| 30 % < Paraxylène PX dans la charge ≤ 45 % | | |
|---|---|---|
| | *t_cycle pond* (1/m) | $Q_D/Q_{PX}$ |
| Réglage n°1 | 325 +/- 25 | 4,40 +/- 0,45 |
| Réglage n°2 | 345 +/- 25 | 4,00 +/- 0,40 |
| Réglage n°3 | 365 +/- 25 | 3,75 +/- 0,40 |
| Réglage n°4 | 385 +/- 25 | 3,50 +/- 0,40 |
| Réglage n°5 | 415 +/- 25 | 3,30 +/- 0,35 |
| Réglage n°6 | 445 +/- 25 | 3,15 +/- 0,35 |
| Réglage n°7 | 485 +/- 25 | 3,00 +/- 0,35 |
| Réglage n°8 | 530 +/- 25 | 2,90 +/- 0,35 |
| Réglage n°9 | 580 +/- 30 | 2,80 +/- 0,30 |
| Réglage n°10 | 640 +/- 30 | 2,80 +/- 0,30 |

**Tableau 3 : Paraxylène PX dans la charge : 45 % < %$_{PX,charge}$ ≤ 60 %**

| 45 % < Paraxylène PX dans la charge ≤ 60 % | | |
|---|---|---|
| | *t_cycle pond* (1/m) | $Q_D/Q_{PX}$ |
| Réglage n°1 | 315 +/- 25 | 4,10 +/- 0,50 |
| Réglage n°2 | 335 +/- 25 | 3,65 +/- 0,45 |
| Réglage n°3 | 350 +/- 25 | 3,25 +/- 0,35 |
| Réglage n°4 | 370 +/- 25 | 3,05 +/- 0,35 |
| Réglage n°5 | 400 +/- 25 | 2,80 +/- 0,35 |
| Réglage n°6 | 440 +/- 25 | 2,65 +/- 0,30 |
| Réglage n°7 | 480 +/- 25 | 2,55 +/- 0,30 |
| Réglage n°8 | 525 +/- 25 | 2,45 +/- 0,30 |
| Réglage n°9 | 580 +/- 30 | 2,40 +/- 0,30 |
| Réglage n°10 | 640 +/- 30 | 2,35 +/- 0,30 |

**2.** Procédé de séparation des xylènes en lit mobile simulé selon la revendication 1, dans laquelle la température opératoire est comprise entre 100°C et 250°C, préférentiellement entre 120°C et 180°C, et la pression est comprise entre la pression de bulle du mélange de xylènes constituant la charge et 3 MPa.

**3.** Procédé de séparation des xylènes en lit mobile simulé selon la revendication 1, dans laquelle la teneur en eau dans la charge est comprise entre 70 et 140 ppm, et préférentiellement comprise entre 80 et 120 ppm.

**4.** Procédé de séparation des xylènes en lit mobile simulé selon la revendication 1, dans laquelle le désorbant est le paradiéthylbenzène.

Europäisches
Patentamt

European
Patent Office

Office européen
des brevets

# RAPPORT DE RECHERCHE EUROPEENNE

Numéro de la demande

EP 18 16 9349

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| A | WO 2009/019336 A1 (INST FRANCAIS DU PETROLE [FR]; LEFLAIVE PHILIBERT [FR]; LEINEKUGEL LE) 12 février 2009 (2009-02-12) * revendications * ----- | 1-4 | INV. B01D15/18 C07C7/13 C07C15/08 |
| A | US 2009/326309 A1 (PRIEGNITZ JIM [US] ET AL) 31 décembre 2009 (2009-12-31) * le document en entier * ----- | 1-4 | |
| A | US 5 470 482 A (HOLT RANDALL E [US]) 28 novembre 1995 (1995-11-28) * le document en entier * ----- | 1-4 | |

DOMAINES TECHNIQUES RECHERCHES (IPC)

B01D
C07C

Le présent rapport a été établi pour toutes les revendications

| Lieu de la recherche | Date d'achèvement de la recherche | Examinateur |
|---|---|---|
| Munich | 6 septembre 2018 | Fourgeaud, Damien |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
................................................................
& : membre de la même famille, document correspondant

EPO FORM 1503 03.82 (P04C02)

## ANNEXE AU RAPPORT DE RECHERCHE EUROPEENNE
## RELATIF A LA DEMANDE DE BREVET EUROPEEN NO.

EP 18 16 9349

La présente annexe indique les membres de la famille de brevets relatifs aux documents brevets cités dans le rapport de recherche européenne visé ci-dessus.

Lesdits members sont contenus au fichier informatique de l'Office européen des brevets à la date du

Les renseignements fournis sont donnés à titre indicatif et n'engagent pas la responsabilité de l'Office européen des brevets.

06-09-2018

| Document brevet cité au rapport de recherche | Date de publication | Membre(s) de la famille de brevet(s) | Date de publication |
|---|---|---|---|
| WO 2009019336    A1 | 12-02-2009 | BR      PI0813983 A2 | 25-08-2015 |
| | | CN      101765449 A | 30-06-2010 |
| | | CN      104892338 A | 09-09-2015 |
| | | CN      104922935 A | 23-09-2015 |
| | | CN      106064001 A | 02-11-2016 |
| | | FR        2919603 A1 | 06-02-2009 |
| | | JP        5352587 B2 | 27-11-2013 |
| | | JP     2010535178 A | 18-11-2010 |
| | | KR    20100051086 A | 14-05-2010 |
| | | TW       200927704 A | 01-07-2009 |
| | | US     2009018380 A1 | 15-01-2009 |
| | | WO     2009019336 A1 | 12-02-2009 |
| US 2009326309    A1 | 31-12-2009 | CN      102076824 A | 25-05-2011 |
| | | JP     2011526901 A | 20-10-2011 |
| | | KR    20110034601 A | 05-04-2011 |
| | | PL         215943 B1 | 28-02-2014 |
| | | RU     2011103181 A | 10-08-2012 |
| | | US     2009326309 A1 | 31-12-2009 |
| | | WO     2010002590 A2 | 07-01-2010 |
| US 5470482       A | 28-11-1995 | AUCUN | |

EPO FORM P0460

Pour tout renseignement concernant cette annexe : voir Journal Officiel de l'Office européen des brevets, No.12/82

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 2785196

**Littérature non-brevet citée dans la description**

- Principes de l'Adsorption et des Procédés d'Adsorption. John Wiley & Sons, 1984, 326, , 407

- **SILVA, M. S. P. ; MOREIRA, M. A. ; FERREIRA, A. F. P. ; SANTOS, J. C. ; SILVA, V. M. T. M. ; SÁ GOMES, P. ; MINCEVA, M. ; MOTA, J. P. B. ; RO-DRIGUES, A. E.** Adsorbent Evaluation Based on Experimental Breakthrough Curves: Separation of p-Xylene from C8 Isomers. *Chem. Eng. Technol.,* 2012, vol. 35, 1777-1785
- Diffusion dans les nanopores. Wiley, 2012